(19) Europäisches Patentamt
European Patent Office
Office européen des brevets

(11) EP 2 103 254 B1

(12) EUROPEAN PATENT SPECIFICATION

(45) Date of publication and mention
of the grant of the patent:
15.08.2012   Bulletin 2012/33

(51) Int Cl.:
A61B 5/0468 (2006.01)

(21) Application number: 09001816.9

(22) Date of filing: 10.02.2009

(54) **Device and computer-readable storage medium for classifying VS or VES**

Vorrichtung und computerlesbarer Datenträger zur VS- oder VES-Klassifizierung

Dispositif et support de stockage lisible par ordinateur pour classification VS ou VES

(84) Designated Contracting States:
AT BE BG CH CY CZ DE DK EE ES FI FR GB GR HR HU IE IS IT LI LT LU LV MC MK MT NL NO PL PT RO SE SI SK TR

(30) Priority: 18.03.2008  US 37334 P

(43) Date of publication of application:
23.09.2009   Bulletin 2009/39

(73) Proprietor: Biotronik CRM Patent AG
6341 Baar (CH)

(72) Inventors:
• Lian, Jie
  Beaverton, OR 97007 (US)
• Garner, Garth
  Tigard, OR 97224 (US)
• Müssig, Dirk, Dr.
  West Linn, OR 97068 (US)

(74) Representative: Lindner-Vogt, Karin L.
Biotronik SE & Co. KG
Woermannkehre 1
12359 Berlin (DE)

(56) References cited:
EP-A- 1 995 685          WO-A-2004/105871
DE-A1- 10 159 296        US-A1- 2006 161 069

• WILKINS J: "Correlation-based pattern recognition for implantable defibrillators." PROCEEDINGS : A CONFERENCE OF THE AMERICAN MEDICAL INFORMATICS ASSOCIATION / ... AMIA ANNUAL FALL SYMPOSIUM. AMIA FALL SYMPOSIUM 1996, 1996, pages 289-293, XP002521424 ISSN: 1091-8280
• CHANG K C ET AL: "Comparison of similarity measures for clustering electrocardiogram complexes" COMPUTERS IN CARDIOLOGY, 2005 LYON, FRANCE SEPT. 25-28, 2005, PISCATAWAY, NJ, USA,IEEE, 25 September 2005 (2005-09-25), pages 759-762, XP010889949 ISBN: 978-0-7803-9337-0

**Description**

The present invention generally relates to implantable cardiac devices, including pacemakers, defibrillators and cardioverters, which stimulate cardiac tissue electrically to control the patient's heart rhythm. More particularly, the present invention relates to an apparatus for enhancing the sensing performance by means of morphological analysis of the intracardiac electrogram (IEGM) recorded by the implantable cardiac devices. The method disclosed in this invention is also applicable to devices and systems involving cardiac beat classification based on surface ECG analysis.

**BACKGROUND**

Implantable cardiac devices, such as pacemakers, defibrillators and cardiovertors, have been preferred therapies for treating various cardiac diseases, including bradyarrhythmia, tachyarrhythmia, heart failure, etc. Normal operation of these implantable cardiac devices requires reliable sensing of the cardiac electrical activity.

**DESCRIPTION OF THE RELATED ART**

Currently, the sense detection is based on threshold crossing in all implantable pacemakers and ICDs. That is, a sense event is detected when the measured (and usually filtered) IEGM signal amplitude crosses above a predetermined sensing threshold. To minimize the problem of undersensing and oversensing, modem implantable cardiac devices can automatically adjust the sensing threshold (also known as sensitivity) so that it is adaptive to the IEGM signal amplitude.

However, the problems of oversensing and undersensing still exist, because the threshold crossing method alone could not resolve the sensing problems. Moreover, for sensed events, reliable event classification poses another challenge for the implant device.

Generally, the device sensed events could be classified into four classes: (1) normal intrinsic events, including normal atrial depolarization (P wave), and normal ventricular depolarization (QRS complex); (2) abnormal intrinsic depolarization, including ectopic atrial depolarization, ectopic ventricular depolarization, and retrograde P waves; (3) endogenous noise, including T waves, far-field R waves, far-field T waves, depolarization waveform double counting, and any other non-depolarization signal originating from within the heart; and (4) exogenous noise, including myopotentials, electromagnetic interference, lead failure artifact, in-channel and cross-channel pacing artifact, and any other signal originating from outside the heart.

For implantable cardiac devices, appropriate pacemaker timing and device diagnosis all depend on accurate event classifications. Particularly, the main task of event classification is to differentiate intrinsic events (classes 1 and 2) from non-intrinsic events (classes 3 and 4). Further classification of normal intrinsic events (class 1) from abnormal intrinsic events (class 2) is also desired.

Conventionally, the event classification in implantable cardiac devices is solely based on event timing information. As well known in the art, a plural of time intervals are defined (and mostly programmable) in the implantable cardiac device for event classification, such as atrial refractory period, ventricular refractory period, far-field blanking window, post-pace blanking window, and so on. A sense event outside the refractory period would be classified as an intrinsic event, whereas that inside the refractory period or a blanking window would be classified as a non-intrinsic event.

More complex algorithms were developed to improve the classification accuracy. For example, an atrial sense inside the atrial refractory period would be classified as an intrinsic P wave if it were followed by a normal ventricular sense event within a predefined time interval. On the other hand, atrial senses in the atrial refractory period following ventricular paces would be classified as retrograde P waves if the intervals from ventricular paces to the atrial senses are stable.

In another example, a ventricular sense outside the ventricular refractory period would be classified as a normal ventricular depolarization if it were preceded by an atrial event within a predefined time interval, or a ventricular extrasystole otherwise.

Yet in another example, when a device has no atrial sensing (e.g., in VVI mode), a ventricular sense outside the ventricular refractory period would be classified as a normal ventricular depolarization if the current ventricular coupling interval is not shorter than the average of preceding ventricular intervals by a predefined percentage (also called the prematurity index), otherwise a ventricular extra-systole is declared instead. Similar classification of normal atrial depolarization and atrial extra-systole can also be made.

All above event classification methods have the intrinsic limitation that only event timing information is utilized. As a result, the sensitivity and specificity of event classification is limited, and event misclassification is common in implantable cardiac devices. These limitations, on one hand, can potentially cause delay or withhold of appropriate therapies, and on the other hand, can potentially cause delivery of inappropriate therapies.

It is known that the morphology of the cardiac electric signal contains useful information for cardiac event classification. For example, the normal intrinsic cardiac depolarization usually has different morphology than that of the ectopic beat. Various pattern recognition techniques (e.g., neural network, fuzzy logic, etc.) have been developed for cardiac beat classification based on morphological analysis of the surface ECG signals. However, morphological analysis of IEGM is rarely used in the implantable cardiac devices for event classification, mainly due to the high complexity of these algorithms.

Generally, there are two different approaches for morphological analysis. In one approach, the morphology of the signal is characterized by a plural of metrics either directly measured from the signal (e.g., signal amplitude, width, area, slope, threshold crossing, peak polarity, etc.), or indirectly obtained from the transformed signal (e.g., Fourier transform, wavelet transform, symbolic and other nonlinear transforms, etc.). However, the caveat is that, in principle, the waveform morphology is unlikely to be fully characterized by a single or multiple metrics. In other words, metric-based approach usually results in loss of morphological information.

EP1995685 forming state of the art according to Article 54(2) EPC discloses an implantable cardiac device and method for determining its capture state. A template signal and a test signal are generated from the electrogram, which are transformed into sample values by comparison with predetermined template subspaces. An Adaptive Signed Correlation Index (ASCI) between the template signal and test signal is determined to indicate the capture state of the device.

EP2103253 forming state of the art according to Article 54(3) EPC discloses an implantable cardiac device and method for classification of SVT from VT. A template signal and a test signal are generated from the electrogram, which are transformed into sample values by comparison with predetermined template subspaces. An Adaptive Signed Correlation Index (ASCI) between the template signal and test signal is determined to classify SVT from VT.

In another approach, the morphology of two signals can be compared directly by means of correlation analysis. High correlation between two signals indicates they have similar morphology, whereas low correlation indicates they have different morphology. The most commonly used correlation measure is the Pearson's Correlation Coefficient (PCC). However, it has two major limitations. First, the calculation of PCC requires floating point operation, which renders it not feasible for implementation in the low-power embedded systems, particularly the battery-powered implantable cardiac devices. Second, PCC does not account for the amplitude difference between signals and is sensitive to the impulsive noise.

In view of above, there is a need to provide the implantable cardiac device with a novel method to accurately, efficiently, and robustly perform IEGM morphology analysis, to facilitate sense event classification.

It is an object of the invention to provide a device, for example an implantable cardiac device, such as a pacemaker, a defibrillator, a cardioverter or a biventricular pacing device, that can sense cardiac electrical signals and accurately classify the sensed events. The device is defined by claim 1.

The electrogram is a ventricular electrogram (VEGM).,

The device comprises means for constructing a normal ventricular sense (VS) template waveform from the VEGM signal that corresponds to antegrade conducted ventricular depolarization. The device may further comprise means for constructing a normal atrial sense (AS) template waveform from the AEGM signal that corresponds to normal intrinsic atrial depolarisation, for constructing a retrograde AS template waveform from the AEGM signal that corresponds to retrograde atrial depolarization, or combinations thereof. The VS template waveforms can be further constructed for normal right ventricular (RV) sense event, and normal left ventricular (LV) sense event, respectively.

The device may provide means for constructing the normal atrial sense (AS) template waveform from the AEGM corresponding to the AS events detected outside the atrial refractory period (ARP), for constructing the normal ventricular sense (VS) template waveform from the VEGM corresponding to VS event that is outside the ventricular refractory period (VRP) and is associated with preceding AS event or atrial pace (AP) event, for constructing the retrograde AS template waveform from the AEGM corresponding to retrograde AS events, or combinations of such means. For confirming retrograde AS events, the device comprises in a further special embodiment means for determining the stability of intervals from the ventricular paces (VP) to the refractory atrial senses.

The device comprises means for averaging a plurality of cycles of signals to obtain the template signal. Especially cycles of conducted ventricular IEGM signals may be used.

The device comprises means for updating the template periodically or continuously after an initial template setup.

The device comprises means for aligning the signals based on at least one predefined fiducial point.

In another embodiment not part of invention the device comprises means for exposing the sense events, means for collecting multiple cycles of the IEGM signals containing the sense events, means for aligning the IEGM signals based on at least one predefined fiducial point; and means for creating the sense template waveform by averaging similarly aligned IEGM cycles.

The device may further comprising means for generating threshold vectors bounding the subspaces, where the threshold vectors are generated by increasing or decreasing the sample values of the template signal by a predefined fix value, or by a predefined ratio, or by their combinations.

The means for transforming comprises means for setting a sample value of the transformed signal to a first, second or third integer if the corresponding sample value of the signal belongs to the first, second or third subspace. In a special embodiment the first integer is set to 1, the second integer is set to 0 and the third integer is set to -1.

The device comprises means for determining a correlation using at least the transformed test signal.

In yet a further embodiment of the invention the device comprises means for determining a correlation using only the transformed test signal.

The means for determining a correlation comprises means for determining an Adapted Signed Correlation Index (ASCI) as the sum of the sample values of the transformed test signal or by dividing the sum of the sample values of the transformed test signal by the number of samples.

It is a further object of the invention to provide a device which comprises means for detecting ectopic beat events by calculating the correlation between templates and test signals based on morphological analysis, especially based on ASCI-based morphological analysis. The device comprises means for determining correlation between a normal VS template and a test VEGM signal, and means for indicating, depending from the correlation value, a normal intrinsic VS template or a ventricular extra-systole (VES) event. The device may comprise means for determining correlation between a normal AS template and a test AEGM signal, and means for indicating, depending from the correlation value, a normal intrinsic AS event or an atrial extra-systole (AES) event.

The ASCI-based morphological analysis may be combined with at least time interval analysis, and signal processing algorithms for ectopic beat detection in devices or systems (e.g., ECG monitors, ECG Holters, etc.) involving measurement and analysis of surface ECG signals. The device may comprise means for activating the ectopic beat detection only when the time interval analysis could not definitely differentiate normal beat from ectopic beat. However, ectopic beat detection may also be conducted independent from a time interval analysis.

The device may comprise means for differentiating retrograde atrial depolarization from normal intrinsic atrial depolarization by calculating the correlation between templates based on morphological analysis, especially based on ASCI-based morphological analysis. The device for differentiating retrograde atrial depolarization from normal intrinsic atrial depolarization comprises means for calculating for an AS event detected within the post-ventricular atrial refractory period (PVARP) a first correlation value by comparing the corresponding AEGM with a normal AS template, means for indicating, depending from the first correlation value, the event as a normal intrinsic AS or not, and then calculating a second correlation value by comparing the corresponding AEGM with a retrograde AS template, and means for indicating, depending from the second correlation value, the event as a retrograde AS or neither a normal intrinsic AS, nor a retrograde AS. In a preferred embodiment, the device is arranged in such a way that an event is ignored, if the event is indicated as neither a normal intrinsic AS, nor a retrograde AS.

The device for differentiating retrograde atrial depolarization from normal intrinsic atrial depolarization may be combined with a time interval analysis.

The device may comprise means for far field sensing classification by calculating the correlation between templates based on morphological analysis, especially based on ASCI-based morphological analysis. The device for far field sensing may comprise means for calculating for an AS event detected within a far-field blanking (FFB) window a first correlation value by comparing the corresponding AEGM with a normal AS template, means for indicating, depending from the first correlation value, the event as a normal intrinsic AS or not, then further checking whether the AS event detected within the FFB is preceded by a VP or a VES event, means for calculating, depending from the result of the check, a second correlation value by comparing the corresponding AEGM with a retrograde AS template, and means for indicating, depending from the second correlation value, the event as a retrograde AS or neither a normal intrinsic AS, nor a retrograde AS. The device for far field sensing may be arranged that an AS event in FFB is ignored, if the event is not preceded by a VP or a VES event, or if the event is indicated as neither a normal intrinsic AS, nor a retrograde AS.

The device for far field sensing may comprise at least one of means for calculating a correlation value by comparing the corresponding RV IEGM with the normal RV sense template, if an RV sense event is detected within the FFB of a preceding LV event, and indicating, depending from the correlation value, the RV sense event as a normal intrinsic RV sense or as a VES, a far-field sense, or a noise sense, and similarly, calculating a correlation value by comparing the corresponding LV IEGM with the normal LV sense template, if an LV sense event is detected within the FFB of a preceding RV event, and indicating, depending from the correlation value, the LV sense event as a normal intrinsic LV sense or as a VES, a far-field sense, or a noise sense. Such a device may be arranged for ignoring the RV or the LV sense event if it is indicated as VES, far-field sense, or noise sense.

Preferably, the device for far field sensing is also combined with a time interval analysis.

It is further an objective of the invention to provide a computer-readable storage medium storing program code for causing a data processing device to perform a method for classifying sense events in implantable devices using signals provided by an electrogram as defined by claim 4.

According to this invention, a novel index called Adaptive Signed Correlation Index (ASCI) is used to quantify the morphology similarity between two IEGM waveforms. The calculation of ASCI is computationally efficient because no floating-point operation is necessary. Furthermore, calculation of ASCI is robust against measurement noise and minor alignment error of the signals.

In a typical embodiment, template signals are created and maintained to represent the normal atrial depolarization and normal ventricular depolarization, respectively. Preferably, the template waveforms are created by averaging multiple cycles of IEGM signals (all aligned with predefined fiducial point) representing normal intrinsic depolarization. The template waveforms are also preferably updated periodically or dynamically to reflect the gradual change of the IEGM morphology.

Upon a sensed atrial event or ventricular event, the corresponding IEGM waveform is compared to the respective template signal, and their similarity is quantified by the ASCI. High ASCI value that is greater than a predefined threshold indicates normal intrinsic cardiac activity, whereas low ASCI value that is lower than the predefined threshold indicates non-intrinsic cardiac activity or abnormal cardiac activity. Combined with time interval analysis, the ASCI-based morphology analysis improves accuracy of the event classification, thus enhancing the sensing function of the device.

The details of the invention can be understood from the following drawings and the corresponding text descriptions.

Fig. 1     is a high-level flowchart diagram that illustrates the steps involved in automatic setup of the IEGM template in an implantable cardiac device.

Fig. 2     shows an example of template setup for normal AS events and normal VS events.

Fig. 3     shows a high-level flowchart that illustrates the steps involved in running update of the IEGM template in an implantable cardiac device.

Fig. 4     illustrates the concept of signal alignment using multiple fiducial points.

Fig. 5     illustrates the concept of three subspaces defined by four threshold vectors that are adaptive to the template signal.

Fig. 6     shows four examples of calculating ASCI for particular application to atrial and ventricular ectopic beat detection in an implantable cardiac device.

Construction of Template Waveform

A normal atrial sense (AS) template waveform may be constructed from the atrial electrogram (AEGM) signal that corresponds to normal intrinsic atrial depolarization. For implantable cardiac devices, the normal atrial template waveform can be constructed from the AEGM corresponding to the AS events detected outside the atrial refractory period (ARP). As known in the art, the normal AS events can be exposed by programming the device atrial pacing rate below the sinus rate of the heart.

According to this invention, a normal ventricular sense (VS) template waveform is constructed from the ventricular electrogram (VEGM) signal that corresponds to antegrade conducted ventricular depolarization. For implantable cardiac devices, the normal VS template can be constructed from the VEGM corresponding to VS event that is outside the ventricular refractory period (VRP) and is associated with preceding AS event or atrial pace (AP) event. As known in the art, the normal VS events can be exposed by programming device atrio-ventricular (AV) delay longer than the intrinsic AV conduction time.

A retrograde AS template waveform may be constructed from the AEGM signal that corresponds to retrograde atrial depolarization. For implantable cardiac devices, the retrograde AS template can be constructed from the AEGM corresponding to retrograde AS events. As known in the art, the retrograde AS events can be exposed by programming device in VDI mode, programming ventricular pacing rate higher than the intrinsic heart rate, and programming a long post-ventricular atrial refractory period (PVARP). The retrograde AS events can be confirmed by relatively stable interval from the ventricular paces (VP) to the refractory atrial senses.

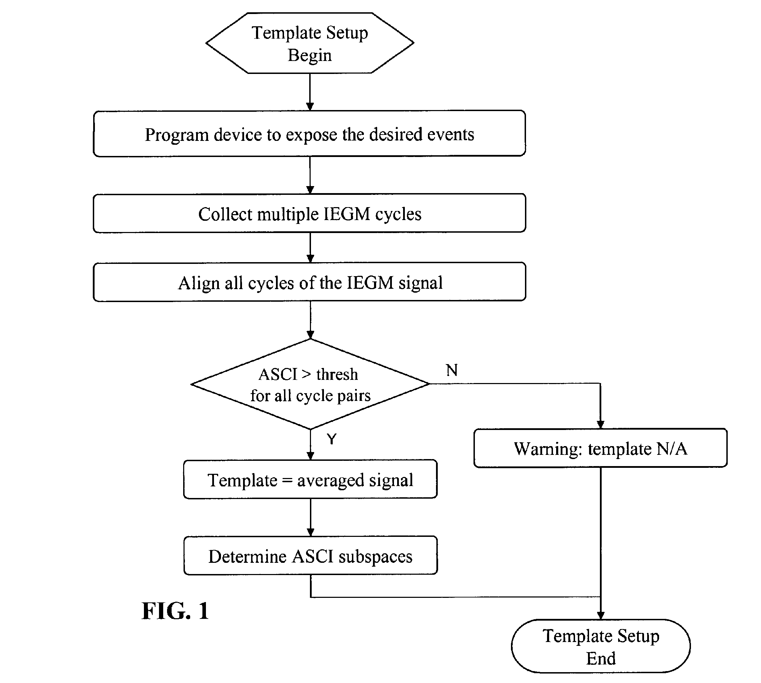

Figure 1 shows a high-level flowchart diagram that illustrates the steps involved in automatic setup of the IEGM templates in an implantable cardiac device. The IEGM templates can be normal AS template, normal VS template, or retrograde AS template. Preferably, the automatic template setup is performed during initial device implant, or during regular device follow-up, when the physician can verify the template signal is constructed from valid IEGM signals.

As described above, to construct the desired IEGM templates (normal AS template, normal VS template, retrograde AS template), the device is programmed accordingly in order to expose the desired sense events (normal AS events, normal VS events, retrograde AS events). Then the device collects multiple cycles of the IEGM signals containing the desired sense events (normal AS, normal VS, retrograde AS). The IEGM signals are then aligned based on predefined fiducial point, for example, the positive or negative peak, the maximum slope, the threshold crossing point, etc., as known in the art. For each cycle, the IEGM segment in a fixed window relative to the fiducial point is selected for creating the template signal. In a typical embodiment, the fiducial point is chosen as the dominant peak (positive or negative) of the IEGM signal, and the IEGM window spans from 50 ms before the fiducial point to 100 ms after the fiducial point.

Still refer to Figure 1. According to this invention, for each pair of the aligned and windowed IEGM signals, their morphological similarity is quantified by an Adaptive Signed Correlation Index (ASCI), which will be described in details in the following sections. If for any given cycle pair, the calculated ASCI is lower than a predefined threshold value, then the collected IEGM signals are considered not stable. A warning is generated by the device indicating the template signal is not available at the moment, and the template setup may be retried at a later time. On the other hand, if for all cycle pairs, the calculated ASCI is greater than the predefined threshold value (e.g., 0.8), then all collected IEGM cycles are considered similar, and the desired IEGM template is created by averaging all these aligned IEGM cycles.

As discussed in more details later, the ASCI is calculated based on the definition of three subspaces that are dependent on the template signal. Thus upon creation of the IEGM template, the device further determines the three subspaces as discussed thereinafter. Note that during the initial template setup phase when template waveform has not been available yet, to calculate the ASCI between a pair of IEGM cycles, any one of the two IEGM signals can be initialized as the tentative template signal. Based on this tentative template signal, the three subspaces can be defined, and the similarity between these two signals can be quantified by ASCI. As discussed above, only if all pairs of the collected IEGM cycles result in higher than predefined ASCI threshold, then these cycles are considered to have similar morphology, and the true template waveform can be created.

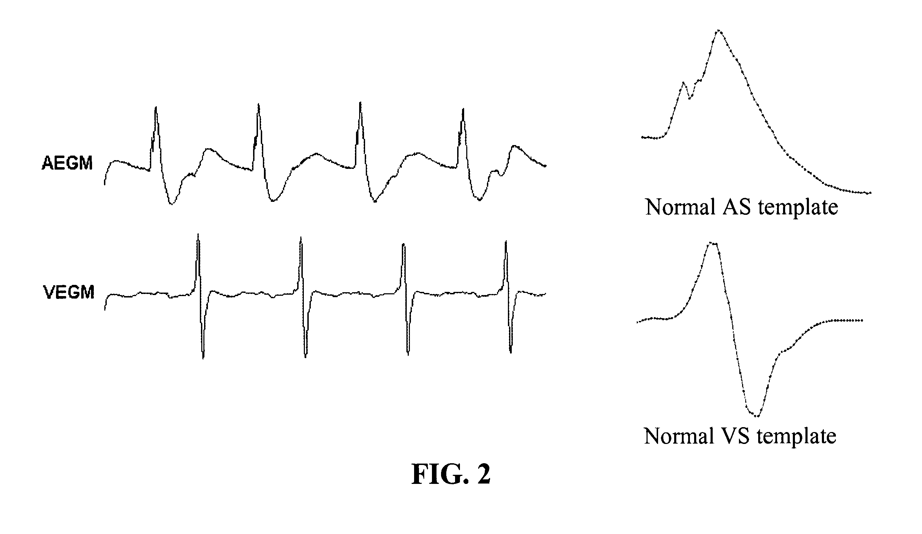

Figure 2 shows a particular example of setting up normal AS template and normal VS template. In this example, the AEGM and the VEGM are shown in the left for four cardiac cycles in sinus rhythm. Each intrinsic atrial depolarization is followed by a conducted ventricular depolarization. Both AEGM and VEGM morphology are consistent among the four cycles. For both AEGM and VEGM, the positive peak is chosen as the fiducial point, and the window size is set from 50 ms before the positive peak to 100 ms after the positive peak. Then the four cycles of AEGM are averaged to construct the normal AS template and the four cycles of VEGM are averaged to construct the normal VS template as shown in the right.

Update of Template Waveform

After the initial template setup, the normal AS template, the normal VS template, and the retrograde AS template are preferably updated periodically or continuously to reflect the dynamic change of the corresponding IEGM morphology. This template running update feature is important because the normal AS, normal VS, and retrograde AS waveforms may gradually change over time due to different factors such as heart rate variation, circadian pattern, changes of medication, changes of electrode-tissue interface, etc.

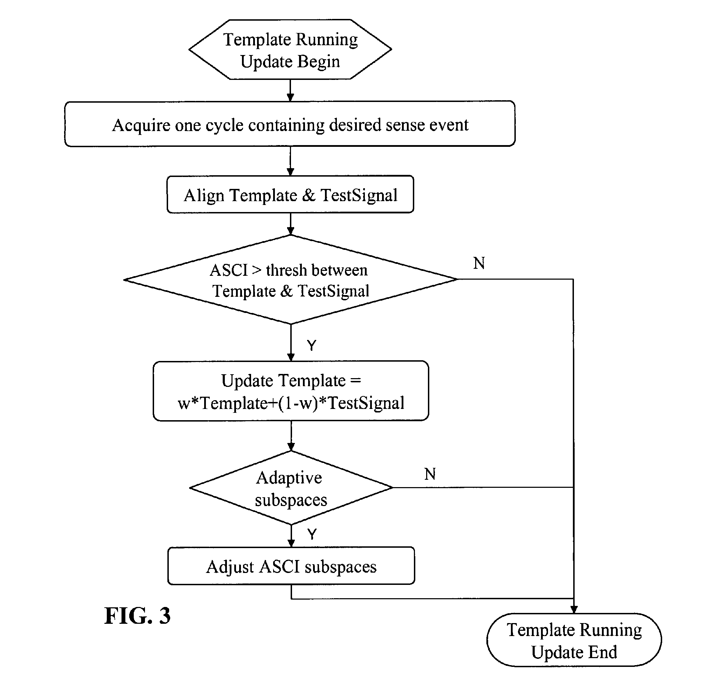

Figure 3 shows a high-level flowchart that illustrates the steps involved in running update of the IEGM templates (normal AS template, normal VS template, retrograde AS template). Preferably, the template running update is activated only when a detected sense event is considered likely to be the desired event type. For example, a detected AS event outside the ARP is likely a normal AS event, a detected VS event outside the VRS and preceded by an AS or AP event is likely a normal VS event, and a detected AS event following a VP event and inside the PVARP is likely a retrograde AS event.

Upon detection of the likely desired sense event (normal AS, normal VS, or retrograde AS), the device acquires one cycle of the corresponding AEGM (for normal AS or retrograde AS) or VEGM (for normal VS) as the test signal, which is aligned with the corresponding template signal (normal AS template, normal VS template, retrograde AS template) based on predefined fiducial point as discussed above.

Then the device calculates the ASCI between the acquired test signal and the corresponding template signal. If the ASCI is lower than a predefined threshold (e.g., 0.8), then the test signal is considered different than the template signal, and no template update is performed for this test cycle. On the other hand, if the calculated ASCI is greater than the predefined threshold (e.g., 0.8), then the test signal is considered similar to the template signal, and the template signal is updated by taking the weighted average of the original template signal and the newly acquired test signal. In an exemplary embodiment, the new template is the sum of the old template signal scaled by 255/256, and the newly acquired test signal scaled by 1/256. By this means, it ensures the stability of the template waveform by retaining 255/256 of the old template signal, whereas it incorporates 1/256 of the test signal to factor in any gradual change of the IEGM morphology of the corresponding event type.

As discussed in more details later, the ASCI is calculated based on the definition of three subspaces which are dependent on the template signal. Thus the device can further adjust the three subspaces based on newly updated template signal, if the adaptive subspace feature is enabled.

Signal Alignment

One prerequisite for any morphology-based event classification algorithms is that the test signal must be properly aligned with the template signal. Morphological analysis based on misaligned signals may yield misleading results. As discussed above, the common practice for signal alignment is based on a predefined fiducial point, such as the positive peak, the negative peak, etc. However, in some cases, the signal alignment based on a single fiducial point is not reliable.

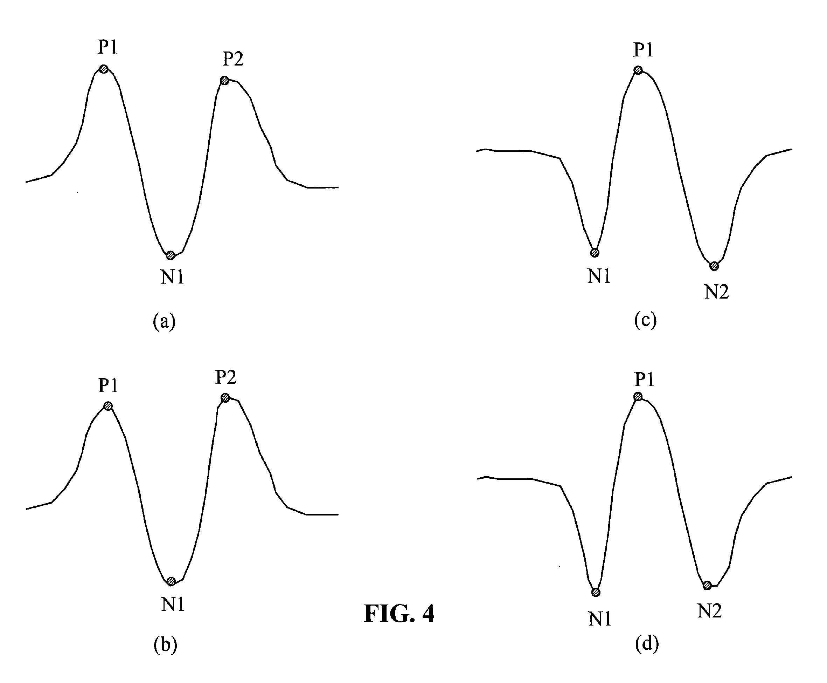

Figure 4 shows some examples. Panels (a) and (b) show two signal complexes that have similar morphology. Both signal complexes can be characterized by two positive peaks (P1, P2) that have similar amplitude and one negative peak (N1). If the dominant positive peak is chosen as the fiducial point, then the fiducial point will be P1 for the signal complex shown in panel (a) but P2 for the signal complex shown in panel (b). Similarly, panels (c) and (d) show another pair of signal complexes that have similar morphology. Both signal complexes can be characterized by two negative peaks (N1, N2) that have similar amplitude and one positive peak (P1). If the dominant negative peak is chosen as the fiducial point, then the fiducial point will be N2 for the signal complex shown in panel (c) but N1 for the signal complex shown in panel (d).

According to this invention, multiple fiducial points are defined for signal alignment in adjunction with ASCI-based morphological analysis. Specifically, for a given template signal representing the desired event type (normal AS, normal VS, retrograde AS), multiple fiducial points (if available) are defined in a sequential order, that is, $1^{st}$ fiducial point, $2^{nd}$ fiducial point, $3^{rd}$ fiducial point, etc. Similar fiducal points (if available) are also identified for a test IEGM signal. For example, for the signals shown in panels (a) and (b) of Figure 4, the fiducial points can be defined in the following order: dominant positive peak ($1^{st}$ fiducial point; P1 in (a) and P2 in (b)), dominant negative peak ($2^{nd}$ fiducial point; N1 in both (a) and (b)), secondary positive peak ($3^{rd}$ fiducial point; P2 in (a) and P1 in (b)). Similarly, for the signals shown in panels (c) and (d) of Figure 4, the fiducial points can be defined in the following order: dominant positive peak ($1^{st}$ fiducial point; P1 in both (c) and (d)), dominant negative peak ($2^{nd}$ fiducial point; N2 in (c) and N1 in (d)), secondary negative peak ($3^{rd}$ fiducial point; N1 in (c) and N2 in (d)).

To compare the morphology of a test signal and the corresponding template signal, the two signals are first aligned with the $1^{st}$ fiducial point, and their ASCI value is calculated. If the resulting ASCI value is higher than a predefined threshold (e.g., 0.8), then it indicates the two signals have similar morphology. The signal alignment is considered valid, and no further calculation is needed. On the other hand, if the resulting ASCI value is lower than the predefined threshold (e.g., 0.8), then it indicates the two signals have different morphology. Then the signals are re-aligned with the $2^{nd}$ fiducial point (if available for both signals), and their ASCI value is re-calculated. If the re-calculated ASCI value is higher than the predefined threshold (e.g., 0.8), then it indicates misalignment for the $1^{st}$ fiducial point, but the alignment based on the $2^{nd}$ fiducial point is valid. The signals are considered to have similar morphology and no further calculation is needed. Similar test can be performed for the $3^{rd}$ fiducial point (if available for both signals) if the ASCI value obtained for the $2^{nd}$ fiducial point is still lower than the predefined threshold (e.g., 0.8). No further test is needed if a fiducial point is only available for one signal but not the other signal. If all ASCI values are below the predefined threshold (e.g., 0.8), no matter which fiducial point is chosen, then it is determined that the test signal and the template signal have different morphology.

According to the experience of the present inventors, using two fiducial points (e.g., dominant positive peak and dominant negative peak) for signal alignment can effectively solve most of the signal misalignment problems caused by using a single fiducial point.

Definition of Adaptive Subspaces

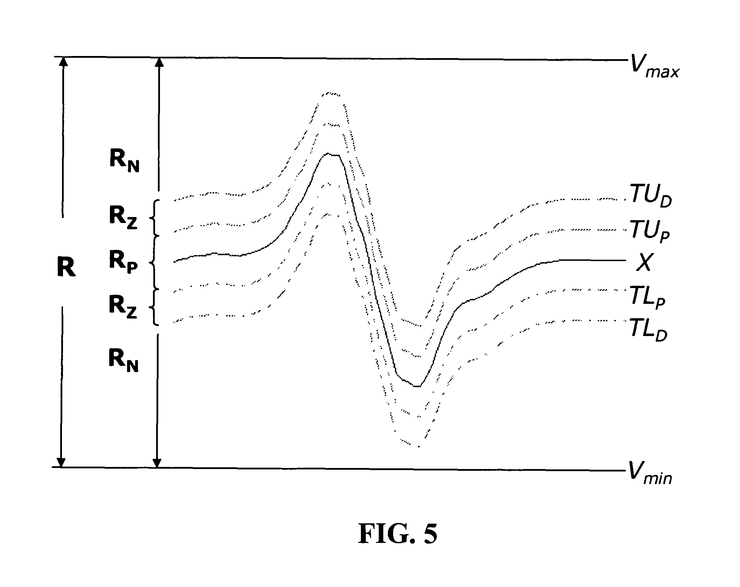

Refer to Figure 5. Let **R** denote the IEGM signal space that spans from $V_{min}$ to $V_{max}$, where $V_{min}$ is the minimum amplitude and $V_{max}$ is the maximum amplitude that could be measured by the sensing channel of the device. Divide **R** into three subspaces $\mathbf{R_P}$, $\mathbf{R_Z}$, and $\mathbf{R_N}$ such that $\mathbf{R} = \mathbf{R_P} \cup \mathbf{R_Z} \cup \mathbf{R_N}$ and $\mathbf{R_P} \cap \mathbf{R_Z} = \mathbf{R_P} \cap \mathbf{R_N} = \mathbf{R_Z} \cap \mathbf{R_N} = \emptyset$, where $\cup$ is the union operator, $\cap$ is the intersection operator, and $\emptyset$ represents the null space. That is, the three subspaces are non-overlapping yet all together they span the whole signal space. For convenient purpose, in the following descriptions, we term $R_P$ as the positive subspace, $\mathbf{R_Z}$ as the zero subspace, and $\mathbf{R_N}$ as the negative subspace.

Still refer to Figure 5. According to this invention, all three subspaces ($\mathbf{R_P}$, $\mathbf{R_Z}$, $\mathbf{R_N}$) are adaptive to the template signal representing IEGM morphology of the desired event type (normal AS, normal VS, or retrograde AS). In a preferred embodiment, four threshold vectors $TL_D$, $TL_P$, $TU_P$, $TU_D$ are defined from the template signal $X$. Denote $X = [x(1), x(2), ..., x(L)]$, where $L$ is the number of samples in signal $X$. Further denote $TL_P = [tlp(1), tlp(2), ..., tlp(L)]$ as the proximal lower threshold vector, $TL_D = [tld(1), tld(2), ..., tld(L)]$ as the distal lower threshold vector, $TUp = [tup(1), tup(2), ..., tup(L)]$ as the proximal upper threshold vector, and $TU_D = [tud(1), tud(2), ..., tud(L)]$ as the distal upper threshold vector. These threshold vectors are defined such that $TL_D \leq TL_P \leq X \leq TU_P \leq TU_D$, or specifically, $tld(i) \leq tlp(i) \leq x(i) \leq tup(i) \leq tud(i)$, for $1 \leq i \leq L$. The positive subspace $\mathbf{R_P}$ is defined as the region bounded by $TL_P$ and $TU_P$, the negative subspace

$R_N$ is defined as the region above $TU_D$ or below $TL_D$, and the zero subspace $R_Z$ is defined as the region bounded between $TU_P$ and $TU_D$, and that between $TL_D$ and $TL_P$. Obviously, a sample in $R_P$ is proximal to the template, a sample in $R_N$ is distal to the template, and a sample in $R_Z$ is at intermediate distance to the template.

According to an exemplary embodiment of the present invention, the four threshold vectors are defined from the template signal according to the following equations:

$$TU_P = X + \alpha \cdot \max(abs(X))$$

$$TL_P = X - \alpha \cdot \max(abs(X))$$

$$TU_D = X + \beta \cdot \max(abs(X))$$

$$TL_D = X - \beta \cdot \max(abs(X))$$

Here, *max(abs(X))* is the peak absolute amplitude of the template signal, $\alpha$ and $\beta$ are programmable scaling coefficients that satisfy $0 < \alpha < \beta$. In a typical example, $\alpha = 0.25$ and $\beta = 0.5$, and the resulting threshold vectors are symmetric around the template signal.

Obviously, there are numerous other means to define the four threshold vectors so that they are adaptive to the template signal *X*, for example, either based on sample-by-sample amplitude *of X,* or based on specific features *of X,* such as its maximum, minimum, max absolute, mean, median, etc., or their combinations. Also, the upper threshold vectors and the lower threshold vectors can be symmetric or asymmetric around the template signal.

As illustrated in Figure 1, after automatic setup of the IEGM template, the three subspaces can be defined from four threshold vectors that are adaptive to the template signal by means of the method described above. Similarly, during the template running update as illustrated in Figure 3, after the template signal is updated by taking the weighted average of the old template signal and the new test signal, the three subspaces can be adjusted by redefining the threshold vectors based on the new template.

Signal Trichotomization

To calculate the ASCI between two IEGM signals, both signals are first trichotomized based on three subspaces that are adaptive to the defined template signal.

Denote **S** as the three-value set {-1 0, 1}. Assume $X = [x(1), x(2), ..., x(L)]$ is an IEGM signal, that is, $x(i)$ **R** for $i = 1, 2, ... L$, where $L$ is the number of samples in signal $X$. Trichotomization of signal X is an operation that maps the signal from **R** space to **S** space. Specifically, denote $TX = [tx(1, tx(2), ..., tx(L)]$ as the trichotomized signal of $X,$ where $tx(i) \in$ **S** for $i = 1, 2, ... L$. Then the trichotomization is formulated as:

$$tx(i) = \begin{cases} 1 & if \ x(i) \in R_P \\ 0 & if \ x(i) \in R_Z \\ -1 & if \ x(i) \in R_N \end{cases}$$

In other words, signal $X$ is trichotomized to $TX$ by converting all its data samples to values selected from {-1, 0, 1}, based on which subspace each data sample belongs to.

In a typical embodiment, signal $X$ is the template signal of the desired event type (normal AS, normal VS, or retrograde AS), and *ASCI(X,Y)* measures the morphological similarity between a test IEGM signal $Y$ and the template signal $X$. For the template signal $X,$ all elements of its trichotomized signal $TX$ are 1s because all samples of $X$ are within the positive subspace $R_P$. For another signal $Y$, its trichotomized signal $TY$ will have more 1s if more samples of $Y$ are close to the corresponding samples of $X,$ i.e., $Y$ is similar to $X$. As $Y$ gradually deviates from $X,$ its trichotomized signal $TY$ has less 1s, more 0s, and eventually more -1s.

Calculation of ASCI

Assume $X=[x(1), x(2), ..., x(L)]$ and $Y=[y(1), y(2), ..., y(L)]$ are two signals in **R,** and each has $L$ samples. Given defined subspaces $\mathbf{R_P}$, $\mathbf{R_Z}$, and $\mathbf{R_N}$ (which are adaptive to the template signal), $X$ is trichotomized to $TX=[tx(1), tx(2), ..., tx(L)]$, and $Y$ is trichotomized to $TY=[ty(1), ty(2), ..., ty(L)]$. The *ASCI between X and Y*, or *ASCI(X, Y),* which measures the similarity between $X$ and $Y$, is defined by the following formula:

$$ASCI(X,Y) = \frac{TX \circ TY}{\sqrt{TX \circ TX} \cdot \sqrt{TY \circ TY}}$$

Here, the symbol denotes the signed correlation (SC) of two trichotomized vectors, and is defined by the following formula:

$$TX \circ TY = \sum_{i=1}^{L} tx(i) \otimes ty(i)$$

Here, the symbol $\otimes$ denotes the signed product (SP) between two trichotomized scalars, and is defined by the following formula:

$$tx(i) \otimes ty(i) = \begin{cases} 1 & if \ tx(i) = ty(i) \\ -1 & if \ tx(i) \cdot ty(i) = -1 \\ 0 & otherwise \end{cases}$$

Accordingly, *if tx(i) = ty(i),* their SP is 1. In this case, the sample pair $x(i)$ and $y(i)$ are considered concordant, meaning that they are in the same subspace. Specifically, both are in the positive subspace *if tx(i) = ty(i)* = 1; or both are in the negative subspace *if tx(i) = ty(i)* = -1; or both are in the zero subspace *if tx(i) = ty(i)* = 0.

On the other hand, if *tx(i)·ty(i)*= -1, their SP is -1. In this case, the sample pair x(i) and *y(i)* are considered discordant. Specifically, it occurs when *tx(i)* = 1 and *ty(i)* = -1, or *tx(i)* = -1 and *ty(i)* = 1. In both cases, one sample is in the positive subspace whereas the other sample is in the negative subspace.

Otherwise, the case must be either *tx(i)* = 0 and *ty(i)* $\neq$ 0, or *tx(i)* $\neq$ 0 and *ty(i)* = 0, and their SP is 0. In this case, the sample pair *x(i)* and *y(i)* are considered neither concordant, nor discordant. Specifically, one sample is within the zero subspace, and the other sample is either in the positive subspace or in the negative subspace.

According to the above definition, the SC of two trichotomized vectors ($TX·TY$) is the sum of the SP of all sample pairs $tx(i) \otimes ty(i)$, for $i = 1...L$. Therefore, the SC of two trichotomized signals will be increased by each pair of concordant samples (+1), decreased by each pair of discordant samples (-1), and not affected otherwise (neither concordant nor discordant sample pair).

For two identical signals, all corresponding sample pairs are concordant. Therefore, for above defined $TX$ and $TY,$ it is evident that $TX^oTX=L$ and $TY·TY=L.$ Consequently, the formula for calculating $ASCI(X, Y)$ defined above can be simplified to:

$$ASCI(X,Y) = \frac{TX \circ TY}{L}$$

As discussed above, in a typical embodiment, signal $X$ is the template signal of the desired sense event (normal AS, normal VS, or retrograde AS), and all elements of its trichotomized signal $TX$ are 1 because all samples *of X* are within the positive subspace. Thus, the formula for calculating *ASCI(X, Y)* defined above can be further simplified to:

$$ASCI(X,Y) = \frac{\sum_{i=1}^{L} ty(i)}{L}$$

In other words, the *ASCI(X,Y)* can be simply calculated as the cumulative sum of all trichotomized samples of test signal *Y* normalized by the number of samples.

Properties of ASCI

The definition *of ASCI* is compatible to the conventional definition of Pearson's correlation coefficient *(PCC)*. Similar to *PCC, ASCI* is a normalized index ranging from -1 to +1. If signals *X* and *Y* have similar morphology, they will have more concordant sample pairs, and *ASCI(X, Y)* will approach +1. On the other hand, if signals *X* and *Y* have different morphology, they will have fewer concordant sample pairs, and *ASCI(X, Y)* will be less. If most sample pairs of *X* and *Y* are discordant, then *ASCI(X,Y)* will approach -1. However, *ASCI* is advantageous compared to *PCC* due to at least three reasons:

First, the calculation of *PCC* requires extensive floating-point operation including multiplication, division, and square root. On the other hand, the calculation of *ASCI* only requires comparison and summation. The threshold vectors that are used to define subspaces can be automatically determined from the template signal, through simple operations such as scaling (bit shifting), adding/subtracting, thresholding, etc. The normalization operation (divided by *L*) can be omitted because the total number of samples (*L*) is a known constant. For the purpose of sense event classification in implantable cardiac devices, the *ASCI* will be mainly used for comparison with predefined or user-programmable threshold to determine if two IEGM signals have similar morphology. In this case, the threshold can be defined in the form of X-out-of-Y criterion, or by means of bit shifting operation (e.g., to obtain *L/2, 3L/4,* 7*L*/8, etc.). Therefore, the calculation of *ASCI* is computationally much more efficient, and can be easily implemented in firmware or hardware of the implantable cardiac device.

Second, *PCC* is a parametric measure of linear relationship, and it does not account for the amplitude difference between signals. On the other hand, the calculation of *ASCI* takes amplitude information into consideration. For the examples shown in Figure 5 where the subspaces are defined by four threshold vectors which are adaptive to the template signal *X*, a high *ASCI(X,Y)* value requires *X* and *Y* must stay close and have similar amplitude throughout the signal length (i.e., *Y* must be bounded by proximal upper and lower threshold vectors around *X*); otherwise, low *ASCI (X, Y)* value is obtained.

Thirdly, *PCC* is affected by each sample amplitude of each signal, thus is sensitive to additive noise such as impulse noise or continuous random noise, as well as sensitive to slight yet normal signal variation. On the other hand, the *ASCI(X, Y)* is calculated based on trichotomized signals *TX* and *TY*, and signal trichotomization is further based on subspaces **R<sub>P</sub>, R<sub>Z</sub>,** and **R<sub>N</sub>** that are adaptive to the template signal. Different means to define these subspaces can provide different degrees of tolerance of signal variation. Thus a noise-free signal and the same signal added with noise could have identical trichotomized vectors. Therefore, by properly designing subspaces according to specific application and/or prior knowledge of the signal, the *ASCI* can be more tolerant to additive noise and normal signal variation than *PCC*.

Ectopic Beat Detection

As described above, initial classification of a sense event in the implantable cardiac device can be made based on event timing information. However, misclassification between normal beat and ectopic beat may occur. For example, an AS outside the ARP may represent a normal intrinsic depolarization, or an atrial extra-systole (AES). In another example, an AS inside the ARP that is followed by a VS may also be a normal intrinsic depolarization, or an AES. Yet in another example, a VS outside the VRP may represent an antegrade conducted ventricular depolarization, or a ventricular extra-systole (VES). To improve the classification accuracy, the ASCI-based morphological analysis can be used to facilitate the ectopic beat detection in implantable cardiac devices.

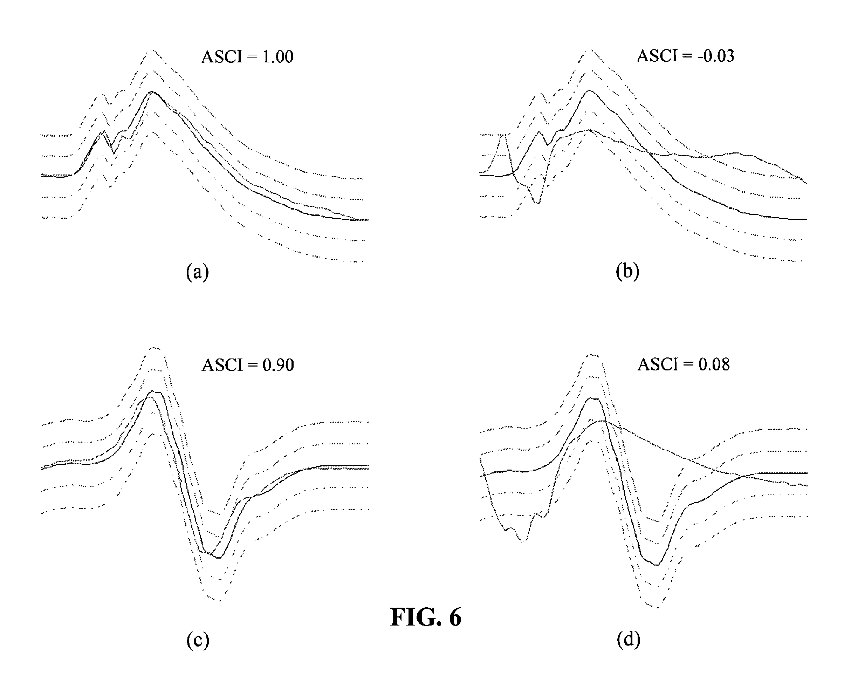

Now refer to Figure 6, which shows four examples of calculating ASCI for particular application to ectopic beat detection in an implantable cardiac device. In these examples, traces labeled with B are the template signals representing the normal AS template (panel (a) and panel (b)) or the normal VS template (panel (c) and panel (d)), and the traces labeled with R are the test AEGM signals corresponding to AS events (panel (a) and panel (b) or the test VEGM signals corresponding to VS events (panel (c) and panel (d)). All template signals and the test signals are aligned based on predefined fiducial points as described above.

In each plot, the four threshold vectors are defined based on the corresponding template signal according to the method illustrated in Figure 5. Then the test signal is trichotomized, and the corresponding ASCI values are calculated.

In panel (a), the calculated ASCI is 1.00, whereas in panel (b), the resulting ASCI is -0.03. Assuming a predefined ASCI threshold of 0.50, then the supra-threshold ASCI obtained in panel (a) indicates the test AEGM has similar morphology as the normal AS template, thus indicating a normal intrinsic AS event. Contrarily, the sub-threshold ASCI obtained in panel (b) indicates the test AEGM has different morphology than the normal AS template, thus indicating an AES event. In panel (c), the calculated ASCI is 0.90, whereas in panel (d), the resulting ASCI is 0.08. Assuming a predefined ASCI threshold of 0.50, then the supra-threshold ASCI obtained in panel (c) indicates the test VEGM has similar morphology as the normal VS template, thus indicating a normal intrinsic VS event. Contrarily, the sub-threshold ASCI obtained in panel (d) indicates the test VEGM has different morphology than the normal VS template, thus indicating a VES event.

Preferably, the ASCI-based morphology analysis is used in conjunction with the time interval analysis for ectopic beat detection. According to one typical embodiment of the present invention, the ASCI-based morphology analysis for ectopic beat detection is only activated when the time interval analysis could not definitely differentiate normal beat from ectopic beat. For example, a VS event without a preceding AS or AP event can be classified as a VES with high confidence, thus no additional morphological analysis is necessary.

Yet according to another embodiment of the present invention, the ASCI-based morphology analysis conducts the ectopic beat detection, independent of the time interval analysis. The analysis results from both methods are combined, e.g., through fuzzy logic, to reach the final classification results.

Yet according to a further embodiment of the present invention, the ASCI-based morphology analysis could be used in conjunction with other signal processing algorithms for ectopic beat detection in devices or systems (e.g., ECG monitors, ECG Holters, etc.) involving measurement and analysis of surface ECG signals.

Retrograde P Wave Classification

Retrograde P wave represents atrial depolarization caused by retrograde AV conduction after VP or VES. As known in the art, to differentiate retrograde atrial depolarization from normal intrinsic atrial depolarization, an implantable cardiac device usually starts a PVARP window after a VP or VES event. An AS event detected outside PVARP can be classified as intrinsic AS event, whereas an AS event detected inside PVARP can be classified as retrograde AS event. However, this simple method often leads to misclassification between intrinsic AS and retrograde AS. In one example, false classification of retrograde AS to intrinsic AS may result in pacemaker mediated tachycardia in devices operating in DDD mode. In another example, false classification of intrinsic AS to retrograde AS may result in delayed or failed mode switch during atrial tachycardia.

Improved event classification algorithms have been implemented in some implantable cardiac devices. The general concept of these algorithms is that an AS event inside the PVARP is likely as an intrinsic P wave if it is followed by a VS event within a predefined time interval, whereas an AS event inside the PVARP is suspected as a retrograde AS if the interval from VP to AS is consistent over multiple cycles. However, the classification accuracy of these algorithms is still limited.

To improve the classification accuracy between intrinsic AS and retrograde AS, the ASCI-based morphological analysis can be used in implantable cardiac devices. The basic concept is that the normal intrinsic atrial depolarization generally has distinct AEGM morphology than that of the retrograde atrial depolarization, due to different atrial activation pathways. Preferably, the ASCI-based morphology analysis is used in conjunction with the time interval analysis for retrograde P wave detection.

For an AS detected within the PVARP, a first ASCI value is calculated by comparing the morphology of the corresponding AEGM with that of the normal AS template. If the obtained first ASCI value is greater than a predefined threshold (e.g., 0.5), then the two signals are considered to have similar morphology, thus suggesting the event is a normal intrinsic AS. Otherwise, the two signals are considered to have different morphology. In such a case, a second ASCI value is calculated by comparing the morphology of the corresponding AEGM with that of the retrograde AS template. If the obtained second ASCI value is greater than a predefined threshold (e.g., 0.5), then the two signals are considered to have similar morphology, thus suggesting the event is a retrograde AS. Otherwise, the two signals are considered to have different morphology. This suggests that the detected AS event is neither a normal intrinsic AS, nor a retrograde AS. This could happen when the detected AS event is an AES, which could be confirmed if it is followed by a VS event within a predefined time interval. Or this could happen when the detected AS event is a far-field sense or a noise sense, and in both cases, the AS event is preferably ignored by the device.

Far-Field Sense Classification

Far-field sensing in implantable cardiac devices can adversely affect arrhythmia detection and therapy. One common problem is the far-field sensing of the ventricular depolarization (R wave) in the atrial channel. Another common problem is the far-field sensing of the ventricular repolarization (T wave) in the atrial channel. In biventricular pacing devices, there are also problems of far-field sensing of right ventricular (RV) depolarization or repolarization in the left ventricular (LV) channel, or far-field sensing of LV depolarization or repolarization in the RV.

To prevent undesirable far-field sensing in one sense channel, a common method is to start a far-field blanking (FFB) window in the channel upon delivering a pace or detecting an intrinsic depolarization in other channels. The length of the FFB must be properly adjusted so that on one hand, it can cover the cross-channel far-field signal, while on the other hand, it does not blank the in-channel real activation signal. However, the FFB adjustment may not be always effective. In some circumstances, the far-field signal may fall outside the FFB window, causing false classification of the sense event. In other circumstances, intrinsic depolarization may occur inside the FFB window. This could happen due to extra-systole, or due to sub-threshold pacing in the channel that does not capture the chamber.

To improve the classification accuracy of the far-field sense, the ASCI-based morphological analysis can be used in implantable cardiac devices. The basic concept is that the intrinsic cardiac depolarization generally has distinct IEGM morphology than that of the far-field sense signal. Preferably, the ASCI-based morphology analysis is used in conjunction with the time interval analysis for far-field sense classification.

For an AS detected within the FFB, a first ASCI value is calculated by comparing the morphology of the corresponding AEGM with that of the normal AS template. If the obtained first ASCI value is greater than a predefined threshold (e.g., 0.5), then the two signals are considered to have similar morphology, thus suggesting the event is a normal intrinsic AS. Otherwise, the two signals are considered to have different morphology. If the far-field AS event is not preceded by a VP nor a VES event, no further check of retrograde AS is needed. Otherwise (i.e., the far-field AS event is preceded by a VP or a VES event), a second ASCI value is calculated by comparing the morphology of the corresponding AEGM with that of the retrograde AS template. If the obtained second ASCI value is greater than a predefined threshold (e.g., 0.5), then the two signals are considered to have similar morphology, thus suggesting the event is a retrograde AS. Otherwise, the two signals are considered to have different morphology. This suggests that the detected AS event is neither a normal intrinsic AS, nor a retrograde AS. This could happen when the detected AS event is an AES, which could be confirmed if it is followed by a VS event within a predefined time interval. Or this could happen when the detected AS event is a far-field sense or a noise sense, and in both cases, the AS event is preferably ignored by the device.

A normal RV sense template and a normal LV sense template are respectively constructed and maintained in a biventricular pacing device. For an RV sense detected within the FFB of a preceding LV event, an ASCI value is calculated by comparing the morphology of the corresponding RV IEGM with that of the normal RV sense template. If the obtained ASCI value is greater than a predefined threshold (e.g., 0.5), then the two signals are considered to have similar morphology, thus suggesting the event is a normal intrinsic RV sense. Otherwise, the two signals are considered to have different morphology. This could happen when the detected RV sense event is a VES, or a far-field sense, or a noise sense, and in all these cases, the RV sense event is preferably ignored by the device. Evidently, similar event classification can be conducted for an LV sense detected within the FFB of a preceding RV event.

**Claims**

1. A device for classifying sense events in implantable devices, the device comprising:

- means for providing a template signal and a test signal originated from an electrogram, the template signal and the test signal comprising samples;
- means for associating the template signal with three subspaces, wherein the three subspaces are defined by

- a first subspace comprising values which differ from the template signal values at the most by a predefined first value,
- a second subspace comprising value which differ from the template signal values at the least by the predefined first value and at the most by a predefined second value, and
- a third subspace comprising values which differ from the template signal values at the least by the predefined second value;

- means for transforming the test signal by assigning a first, second or third integer to a sample of the test signal if the corresponding sample of the test signal belongs to the first, second or third subspace;
- means for determining an adapted signed correlation index between the template signal and the test signal as the sum of the sample values of the transformed test signal or by dividing the sum of the sample values of the transformed test signal by the number of samples;
wherein
- the electrogram is a ventricular electrogram (VEGM) and
- the template signal is a normal ventricular sense (VS) template waveform from the VEGM signal that corresponds to antegrade conducted ventricular depolarisation and the device is further adapted to
- generate an adapted signed correlation index between a normal VS template and a test VEGM signal , and to indicate depending on the adapted signed correlation index value whether the test signal is a normal intrinsic VS event or a ventricular extra-systole (VES) event.

2. Device according to claim 1, where the device is an implantable cardiac device, such as a pacemaker, a defibrillator, a cardiovertor or a biventricular pacing device.

3. The device according to claim 1, **characterised in that**

- the template signal is obtained by averaging a plurality of cycles of signals;
- after an initial template setup the template is updated periodically or continuously; and/or
- the signals are aligned based on at least one predefined fiducial point.

4. A computer-readable storage medium storing program code for causing a data processing device to perform a method for classifying sense events in implantable devices using signals provided by an electrogram, the method comprising the steps of:

- providing a template signal and a test signal, the template signal and the test signal comprising samples;
- associating the template signal with three subspaces, wherein the three subspaces are defined by

- a first subspace comprising values which differ from the template signal values at the most by a predefined first value,
- a second subspace comprising values which differ from the template signal values at the least by the predefined first value and at the most by a predefined second value, and

- a third subspace comprising values which differ from the template signal values at the least by the predefined second value;
- transforming the test signal by assingning a first, second or third integer to a sample of the test signal if the corresponding sample of the test signal belongs to the first, second or third subspace; - determining an adapted signed correlation index between the template signal and the test signal as the sum of the sample values of the transformed test signal or by dividing the sum of the sample values of the transformed test signal by the number of samples; and
wherein
- the electrogram is a ventricular electrogram (VEGM) and
- the template signal is a normal ventricular sense (VS) template waveform from the VEGM signal that corresponds to antegrade conducted ventricular depolarisation and
- an adapted signed correlation index between a normal VS template and a test VEGM signal is generated, and depending on the adapted signed correlation index value it is indicated whether the test signal is a normal intrinsic VS event or a ventricular extra-systole (VES) event.

**Patentansprüche**

1. Vorrichtung zum Klassifizieren von Wahrnehmungsereignissen in implantierbaren Vorrichtungen, die Vorrichtung umfassend:

- Mittel zum Bereitstellen eines Mustersignals und eines von einem Elektrogramm hervorgehenden Testsignals, wobei das Mustersignal und das Testsignal Abtastwerte umfassen;
- Mittel zum Zuordnen des Mustersignals zu drei Teilräumen, die definiert werden durch

- einen ersten Teilraum, der Werte umfasst, die von den Mustersignalwerten um höchstens einen vorgeben ersten Wert abweichen,
- einen zweiten Teilraum, der Werte umfasst, die von den Mustersignalwerten um zumindest einen vorgegebenen ersten Wert und höchstens einen vorgegebenen zweiten Wert abweichen, und
- einen dritten Teilraum, der Werte umfasst, die von den Mustersignalwerten um zumindest den vorgegebenen zweiten Wert abweichen;

- Mittel zum Umformen des Testsignals durch Zuordnen einer ersten, zweiten oder dritten Ganzzahl zu einem Abtastwert des Testsignals, wenn der entsprechende Abtastwert des Testsignals zum ersten, zweiten oder dritten Teilraum gehört;

- Mittel zum Bestimmen eines angepassten, vorzeichenbehafteten Korrelationsindexes zwischen dem Mustersignal und dem Testsignal als Summe der Abtastwerte des umgeformten Testsignals oder durch Dividieren der Summe der Abtastwerte des umgeformten Testsignals durch die Anzahl der Abtastwerte; wobei

- das Elektrogramm ein ventrikuläres Elektrogramm (VEGM) ist und

- das Mustersignal eine normale Musterwellenform einer ventrikulären Wahrnehmung (VS) aus dem VEGM-Signal ist, das einer antegrad durchgeführten Depolarisation entspricht, und die Vorrichtung weiterhin dazu angepasst ist,

- einen angepassten vorzeichenbehafteten Koorelationsindex zwischen einem normalen VS-Muster und einem VEGM-Testsignal zu erzeugen und in Abhängigkeit des angepassten, vorzeichenbehafteten Korrelationsindexwerts anzuzeigen, ob das Testsignal ein normales intrinsisches VS-Ereignis oder ein ventrikuläres Extrasystolenereignis (VES) ist.

2. Vorrichtung nach Anspruch 1, wobei die Vorrichtung eine implantierbare, kardiale Vorrichtung ist wie etwa ein Herzschrittmacher, ein Defibrillator, ein Cardioverter oder eine biventrikuläre Schrittmachervorrichtung.

3. Die Vorrichtung nach Anspruch 1, **dadurch gekennzeichnet dass**

- das sich das Mustersignal durch Mittelung mehrerer Signalzyklen ergibt;
- nach einer erstmaligen Einrichtung des Musters, das Muster periodisch oder fortlaufend aktualisiert wird; und/oder
- die Signale basierend auf zumindest einem Referenzpunkt ausgerichtet sind.

4. Ein Speicherprogrammcode für ein computerlesbares Speichermedium, der eine Datenverarbeitungsvorrichtung dazu veranlasst ein Verfahren zum Klassifizieren von Wahrnehmungsereignissen in implantierbaren Vorrichtungen unter Verwendung von durch ein Elektrogramm bereitgestellten Signalen durchzuführen, wobei das Verfahren die folgenden Schritte umfasst:

- Bereitstellen eines Mustersignals und eines Testsignals, wobei das Mustersignal und das Testsignal Abtastwerte umfassen;
- Zuordnen des Mustersignals zu drei Teilräumen, die definiert werden durch

- einen ersten Teilraum, der Werte umfasst, die von den Mustersignalwerten um höchstens einen vorgegeben ersten Wert abweichen,
- einen zweiten Teilraum, der Werte umfasst, die von den Mustersignalwerten um zumindest einen vorgegebenen ersten Wert und höchstens einen vorgegebenen zweiten Wert abweichen, und

- einen dritten Teilraum, der Werte umfasst, die von den Mustersignalwerten um zumindest den vorgegebenen zweiten Wert abweichen;
- Umformen des Testsignals durch Zuordnen einer ersten, zweiten oder dritten Ganzzahl zu einem Abtastwert des Testsignals, wenn der entsprechende Abtastwert des Testsignals zum ersten, zweiten oder dritten Teilraum gehört;
- Bestimmen eines angepassten, vorzeichenbehafteten Korrelationsindexes zwischen dem Mustersignal und dem Testsignal als Summe der Abtastwerte des umgeformten Testsignals oder durch Dividieren der Summe der Abtastwerte des umgeformten Testsignals durch die Anzahl der Abtastwerte; und wobei

- das Elektrogramm ein ventrikuläres Elektrogramm (VEGM) ist und
- das Mustersignal eine normale Musterwellenform einer ventrikulären Wahrnehmung (VS) aus dem VEGM-Signal ist, das einer antegrad durchgeführten Depolarisation entspricht, und
- ein angepasster vorzeichenbehafteter Korrelationsindex zwischen einem normalen VS-Muster und einem VEGM-Testsignal erzeugt wird und in Abhängigkeit des angepassten vorzeichenbehafteten Korrelationswert angezeigt wird, ob das Testsignal ein normales intrinsisches VS-Ereignis oder ein ventrikuläres Extrasystolenereignis (VES) ist.

**Revendications**

1. Dispositif de classification d'événements de détection dans des dispositifs implantables, le dispositif comprenant:

   - des moyens pour fournir un signal de modèle et un signal de test provenant d'un électrogramme, le signal de modèle et le signal de test comprenant des échantillons;
   - des moyens pour associer le signal de modèle à trois sous-espaces, les trois sous-espaces étant définis par:

      - un premier sous-espace comprenant des valeurs qui diffèrent des valeurs de signal de modèle d'au plus une première valeur prédéfinie,
      - un deuxième sous-espace comprenant des valeurs qui diffèrent des valeurs de signal de modèle d'au moins la première valeur prédéfinie et d'au plus une seconde valeur prédéfinie, et
      - un troisième sous-espace comprenant des valeurs qui diffèrent des valeurs de signal de modèle d'au moins la seconde valeur prédéfinie;

   - des moyens pour transformer le signal de test par attribution d'un premier, deuxième ou troisième nombre entier à un échantillon du signal de test si l'échantillon correspondant du signal de test appartient au premier, deuxième ou troisième sous-espace;
   - des moyens pour déterminer un indice de corrélation signée adapté entre le signal de modèle et le signal de test en tant que la somme des valeurs d'échantillon du signal de test transformé ou par division de la somme des valeurs d'échantillon du signal de test transformé par le nombre d'échantillons;
   dans lequel:
   - l'électrogramme est un électrogramme ventriculaire (VEGM) et
   - le signal de modèle est une forme d'onde de modèle de détection ventriculaire (VS) normal provenant du signal VEGM qui correspond à une dépolarisation ventriculaire conduite antérograde et le dispositif est en outre conçu pour:
   - générer un indice de corrélation signée adapté entre un modèle VS normal et un signal VEGM de test, et indiquer, en fonction de la valeur d'indice de corrélation signée adapté, si le signal de test est un événement VS intrinsèque normal ou un événement d'extrasystole ventriculaire (VES).

2. Dispositif selon la revendication 1, le dispositif étant un dispositif cardiaque implantable, tel qu'un stimulateur cardiaque, un défibrillateur, un défibrillateur à synchronisation automatique ou un dispositif de stimulation biventriculaire.

3. Dispositif selon la revendication 1, **caractérisé par le fait que** :

   - le signal de modèle est obtenu par établissement de la moyenne d'une pluralité de cycles de signaux ;
   - après un réglage initial de modèle, le modèle est mis à jour de façon périodique ou continue ; et/ou
   - les signaux sont alignés sur la base d'au moins un point repère prédéfini.

4. Support de stockage lisible par ordinateur stockant un code de programme pour amener un dispositif de traitement de données à mettre en oeuvre un procédé de classification d'événements de détection dans des dispositifs implantables à l'aide de signaux fournis par un électrogramme, le procédé comprenant les étapes consistant à:

   - fournir un signal de modèle et un signal de test, le signal de modèle et le signal de test comprenant des échantillons;
   - associer le signal de modèle à trois sous-espaces, les trois sous-espaces étant définis par:

      - un premier sous-espace comprenant des valeurs qui diffèrent des valeurs de signal de modèle d'au plus une première valeur prédéfinie,
      - un deuxième sous-espace comprenant des valeurs qui diffèrent des valeurs de signal de modèle d'au moins la première valeur prédéfinie et d'au plus une seconde valeur prédéfinie, et

      - un troisième sous-espace comprenant des valeurs qui diffèrent des valeurs de signal de modèle d'au moins la seconde valeur prédéfinie,;
   - transformer le signal de test par attribution d'un premier, deuxième ou troisième nombre entier à un échantillon du signal de test si l'échantillon correspondant du signal de test appartient au premier, deuxième ou troisième sous-espace ; - déterminer un indice de corrélation signée adapté entre le signal de modèle et le signal de test en tant que la somme des valeurs d'échantillon du signal de test transformé ou par division de la somme des valeurs d'échantillon du signal de test transformé par le nombre d'échantillons; et
dans lequel:
- l'électrogramme est un électrogramme ventriculaire (VEGM) et
- le signal de modèle est une forme d'onde de modèle de détection ventriculaire (VS) normal provenant du signal VEGM qui correspond à une dépolarisation ventriculaire conduite antérograde et
- un indice de corrélation signée adapté entre un modèle VS normal et un signal VEGM de test est généré et, en fonction de la valeur d'indice de corrélation signée adapté, il est indiqué si le signal de test est un événement VS intrinsèque normal ou un événement d'extrasystole ventriculaire (VES).

FIG. 1

Template Setup Begin → Program device to expose the desired events → Collect multiple IEGM cycles → Align all cycles of the IEGM signal → ASCI > thresh for all cycle pairs

N → Warning: template N/A → Template Setup End

Y → Template = averaged signal → Determine ASCI subspaces

AEGM

VEGM

Normal AS template

Normal VS template

**FIG. 2**

```
        ┌──────────────────────────┐
        │  Template Running        │
        │  Update Begin            │
        └──────────────────────────┘
                    │
                    ▼
   ┌─────────────────────────────────────────────┐
   │ Acquire one cycle containing desired sense   │
   │ event                                        │
   └─────────────────────────────────────────────┘
                    │
                    ▼
        ┌──────────────────────────┐
        │  Align Template & TestSignal │
        └──────────────────────────┘
                    │
                    ▼
            ◇ ASCI > thresh between ◇ ──── N ──────┐
            ◇ Template & TestSignal  ◇              │
                    │                               │
                    Y                               │
                    ▼                               │
        ┌──────────────────────────┐               │
        │  Update Template =        │               │
        │  w*Template+(1-w)*TestSignal │            │
        └──────────────────────────┘               │
                    │                               │
                    ▼                               │
            ◇ Adaptive ◇ ──── N ──────┐            │
            ◇ subspaces ◇             │            │
                    │                 │            │
                    Y                 │            │
                    ▼                 │            │
        ┌──────────────────────────┐ │            │
        │  Adjust ASCI subspaces    │ │            │
        └──────────────────────────┘ │            │
                    │                 │            │
                    └─────────────────┘            │
                            │                       │
                            ▼                       ▼
                    ┌──────────────────────────┐
                    │  Template Running        │
                    │  Update End              │
                    └──────────────────────────┘
```

**FIG. 3**

FIG. 4

**FIG. 5**

ASCI = 1.00

(a)

ASCI = -0.03

(b)

ASCI = 0.90

(c)

ASCI = 0.08

(d)

**FIG. 6**

EP 2 103 254 B1

**EP 2 103 254 B1**

## REFERENCES CITED IN THE DESCRIPTION

### Patent documents cited in the description

- EP 1995685 A
- EP 2103253 A